# EUROPÄISCHE PATENTANMELDUNG

(11) **EP 3 181 189 A1**
(43) Veröffentlichungstag der Anmeldung: **21.06.2017**
(21) Anmeldenummer: 16199917.2
(22) Anmeldetag: 22.11.2016
(51) Int. Cl.: A61N 1/05, A61N 1/04

(54) **IMPLANTIERBARE ELEKTRODENLEITUNG UND SATZ VON ELEKTRODENLEITUNGS-MODULEN**

(30) Priorität: 15.12.2015 DE 102015121815
(71) Anmelder: BIOTRONIK SE & Co. KG, 12359 Berlin (DE)
(72) Erfinder: KAISER, Dajana, 12247 Berlin (DE); JADWIZAK, Detmar, 15537 Erkner (DE); FRÜNDT, Carsten, 13057 Berlin (DE); HILLEBRAND, Gordon, 12157 Berlin (DE)
(74) Vertreter: Galander, Marcus

(57) **Zusammenfassung**

Implantierbare Elektrodenleitung zur elektrischen Stimulation und/oder zum Abfühlen von Körpergewebe, mit einem distalen Endabschnitt, der mindestens einen Elektrodenpol und/oder einen Sensor aufweist, einem proximalen Endabschnitt, der mindestens einen Geräteanschlusskontakt aufweist, und einem Leitungskörper, der eine elektrische Zuleitung zur Verbindung des Elektrodenpols bzw. Sensors mit dem Geräteanschlusskontakt enthält, dadurch gekennzeichnet, dass der distale Endabschnitt, proximale Endabschnitt und Leitungskörper separate Module sind, wobei das Modul distaler Endabschnitt an seinem proximalen Ende mindestens einen ersten Verbindungskontakt hat, das Modul proximaler Endabschnitt an seinem distalen Ende mindestens einen zweiten Verbindungskontakt hat und das Modul Leitungskörper an seinem distalen Ende einen zum lösbaren Eingriff mit dem ersten Verbindungskontakt ausgebildeten dritten Verbindungskontakt und an seinem proximalen Ende einen zum lösbaren Eingriff mit dem zweiten Verbindungskontakt ausgebildeten vierten Verbindungskontakt hat, und die Module im verbundenen Zustand der Verbindungskontakte die Elektrodenleitung bilden.

## Beschreibung

### Gebiet der Erfindung

Die Erfindung betrifft eine implantierbare Elektrodenleitung zur elektrischen Stimulation und/oder zum Abfühlen von Körpergewebe, mit einem distalen Endabschnitt, der mindestens einen Elektrodenpol und/oder einen Sensor aufweist, einem proximalen Endabschnitt, der mindestens einen Geräteanschlusskontakt aufweist, und einem Leitungskörper, der eine elektrische Zuleitung zur Verbindung des Elektrodenpols bzw. Sensors mit dem Geräteanschlusskontakt enthält. Des Weiteren betrifft sie einen Satz von Elektrodenleitungs-Modulen zur Bildung einer derartigen Elektrodenleitung.

Implantierbare Elektrodenleitungen der genannten Art sind seit Jahrzehnten bekannt und in massenhaftem Einsatz, insbesondere in Verbindung mit Herzschrittmachern oder implantierten Defibrilatroren, aber auch mit anderen medizinelektronischen Implantaten, wie etwa Neurostimulatoren. Es gibt eine große Vielfalt von derartigen Elektrodenleitungen, die an verschiedene Einsatzzwecke angepasst sind und anatomischen Gegebenheiten der zu behandelnden Patienten (bei denen es sich neben Erwachsenen auch um Kinder, im Einzelfall auch um andere Säugetiere handeln kann) angepasst sind.

Diese Vielzahl von Elektrodenleitungen ist für den Anwender mittlerweile kaum noch überschaubar und führt zu Problemen bei der Beschaffung und Lagerhaltung, im Einzelfall aber auch dazu, dass eine zwar unmittelbar verfügbare, für den speziellen Einsatzzweck aber nicht am besten geeignete Elektrodenleitung verwendet wird. Es versteht sich, dass auch die Kostensituation bei einem solch großen Sortiment von Produkten mit ähnlichem Verwendungszweck nicht optimal sein kann.

Aus der EP 2 281 327 B1 ist ein modularer Leitungsverbinder für implantierbare Leitungen bekannt, mit dem das proximale oder distale Ende einer vorhandenen Elektrodenleitung mit einem leitfähigen Element verlängert werden kann. Aus der US 2014/0316502 A1 ist eine Elektrodenleitung mit einem modular aufgebauten Leitungsende, bei der sich eine Vielzahl von Leitern an die Zuleitungen im Leitungskörper anschließen lässt.

Der Erfindung liegt nach obigem die Aufgabe zu Grunde, eine gattungsgemäße implantierbare Elektrodenleitung anzugeben, die an verschiedene Einsatzzwecke und anatomische Gegebenheiten anpassbar ist und somit dazu beiträgt, den mit der Herstellung, Logistik und Lagerhaltung vieler unterschiedlicher Elektrodenleitungen verbundenen Aufwand zu verringern und die hieraus resultierenden Kosten zu senken.

Diese Aufgabe wird durch eine implantierbare Elektrodenleitung mit den Merkmalen des Anspruchs 1 gelöst. Zweckmäßige Fortbildungen des Erfindungsgedankens sind Gegenstand der abhängigen Ansprüche. Es wird des Weiteren ein Satz von Elektrodenleitungs-Modulen zur Bildung der vorgeschlagenen Elektrodenleitung offenbart.

Die Erfindung schließt die Überlegung ein, eine implantierbare Elektrodenleitung insgesamt modular aufzubauen, und zwar vom proximalen Geräteanschluss bis hin zur distalen Leitungsspitze. Spezieller wird ein Aufbau aus drei Grundmodulen vorgeschlagen, und zwar einen den distalen Endabschnitt bildenden Modul, einem den proximalen Endabschnitt bildenden Modul und einem den Leitungskörper zwischen beiden Endabschnitten bildenden Modul.

Genauer gesagt, lehrt die vorliegende Erfindung, dass der distale Endabschnitt, proximale Endabschnitt und Leitungskörper separate Module sind, wobei das Modul distaler Endabschnitt an seinem proximalen Ende mindestens einen ersten Verbindungskontakt hat, das Modul proximaler Endabschnitt an seinem distalen Ende mindestens einen zweiten Verbindungskontakt hat und das Modul Leitungskörper an seinem distalen Ende einen zum lösbaren Eingriff mit dem ersten Verbindungskontakt ausgebildeten dritten Verbindungskontakt und an seinem proximalen Ende einen zum lösbaren Eingriff mit dem zweiten Verbindungskontakt ausgebildeten vierten Verbindungskontakt hat, und die Module im verbundenen Zustand der Verbindungskontakte die Elektrodenleitung bilden.

Die vorgeschlagene Lösung ermöglicht es, implantierbare Elektrodenleitungen für einen breiten Anwendungsbereich und praktisch sämtliche anatomischen Gegebenheiten aus einem überschaubaren Satz von Modulen für die genannten Hauptabschnitte der Elektrodenleitung zu bilden. Hiermit kann die Herstellung sehr vieler verschiedener Arten kompletter Elektrodenleitungen vermieden werden, was insgesamt die Kosten der Bereitstellung medizinischer Elektrodenleitungen senken kann. Des Weiteren wird die Beschaffung und Lagerhaltung implantierbarer Elektrodenleitungen insgesamt vereinfacht, was zu weiteren Kosteneinsparungen führen kann. Schließlich wird die Bereitstellung einer für den jeweiligen Einsatzzweck und die gegebene Anatomie optimal angepassten Elektrodenleitung erleichtert und in gewissen Situationen überhaupt erst ermöglicht, was grundsätzlich zu einer Qualitätserhöhung der Patientenversorgung führen kann.

In einer Ausführung der Erfindung umfasst das Modul distaler Endabschnitt und/oder das Modul proximaler Endabschnitt und/oder das Modul Leitungskörper mehrere Sub-Module. Hierdurch wird eine weiter verbesserte Anpassung der erfindungsgemäßen Elektrodenleitung an die zahlreichen möglichen Therapie- und Diagnosemodi beim Einsatz moderner elektromedizinischer Implantate gewährleistet.

In einer Ausgestaltung umfasst das Modul Leitungskörper mehrere seriell verbindbare Sub-Module, die insbesondere jeweils an einem Ende mindestens einen Verbindungskontakt vom Typ des ersten Verbindungskontakts und am anderen Ende einen Verbindungskontakt vom Typ des dritten Verbindungskontakts haben. Spezieller ist hierbei vorgesehen, dass das Modul Leitungskörper mindestens ein Sub-Modul umfasst, das einen Elektrodenpol, insbesondere eine Ringelektrode, oder eine Schock-Wendel aufweist. Durch diese Ausgestaltung lässt sich auch ein Stimulations- oder Defibrillations-Abschnitt in den mittleren Bereich einer Elektrodenleitung einbauen, ohne dass für diese, relativ selten benötigte Konfiguration eine spezielle komplette Elektrodenleitung entworfen, gefertigt und auf Lager gehalten werden müsste.

In einer weiteren Ausführung der Erfindung umfasst das Modul distaler Endabschnitt mehrere seriell verbindbare Sub-Module, von denen alle, mit Ausnahme des das distale Ende (die Spitze) der Elektrodenleitung bildenden Sub-Moduls, insbesondere an einem Ende einen Verbindungskontakt vom Typ des dritten Verbindungskontakts und einem anderen Ende einen Verbindungskontakt vom Typ des ersten Verbindungskontakts haben. Hierdurch lassen sich in einfacher und kostengünstiger Weise verschiedene Stimulations-Konfigurationen bzw. Stimulations-/Abfühl-Konfigurationen in einem sehr breiten Einsatzbereich realisieren.

In einer weiteren Ausführung umfasst das Modul proximaler Endabschnitt mehrere Sub-Module mit je mindestens einem Geräteanschlusskontakt und einem Verbindungskontakt vom Typ des zweiten Verbindungskontakts, sowie ein Verzweigungs-Modul, das den zweiten Verbindungskontakt trägt und mehrere Verbindungskontakte vom Typ des vierten Verbindungskontakts hat. Hierdurch wird die vorgeschlagene Elektrodenleitung leicht und unaufwändig anpassbar an unterschiedliche Stimulations- und sonstige Gerätekonfigurationen, speziell auch an Konfigurationen, die mehrere Signalquellen bzw. Signalerfassungsgeräte umfassen.

Während die Erfindung bereits in einer Leiter-/Elektrodenpol-Konfiguration mit einem einzigen Elektrodenpol und einer einzigen Zuleitung realisierbar ist, ist aus derzeitiger Sicht eine Ausführung als "mehradrige" Elektrodenleitung praktisch bedeutsamer. In einer solchen Ausführung umfasst das Modul distaler Endabschnitt eine Mehrzahl von ersten Verbindungskontakten, das Modul proximaler Endabschnitt hat eine Mehrzahl von zweiten Verbindungskontakten, und das Modul Leitungskörper hat eine Mehrzahl von dritten Verbindungskontakten und eine Mehrzahl von vierten Verbindungskontakten, und die Anzahl der Verbindungskontakte aller Typen stimmt überein. In einer beispielhaften Konfiguration sind in der Elektrodenleitung vier Zuleitungen vorgesehen, und entsprechend ist die Anzahl aller Verbindungskontakte jeweils 4. Es versteht sich, dass die Elektrodenleitung auch eine andere Zahl an Zuleitungen und entsprechenden Verbindungskontakten der genannten Typen haben kann.

In einer weiteren Ausführung umfasst das Modul Leitungskörper eine Mehrzahl von Leiterseilen, an denen der oder jeder dritte und vierte Verbindungskontakt durch Crimpen oder Laserschweißen angebracht ist/sind. Grundsätzlich ist hierbei das Konfigurieren der endgültigen Elektrodenleitungs-Konfiguration losgelöst vom Produktionsprozess des Leitungskörpers und sogar unmittelbar im Vorfeld der Implantation möglich. In einer Ausgestaltung ist vorgesehen, dass im Modul Leitungskörper nur ein Teil der Leiterseile mit einem dritten und vierten Verbindungskontakt versehen ist. Die verbleibenden Leiterseile bleiben dann ohne elektrischen Anschluss und somit ohne Funktion.

In einer anwendungstechnisch vorteilhaften Ausführung sind die ersten und hierin eingreifenden dritten Verbindungskontakte sowie die zweiten und hierin eingreifenden vierten Verbindungskontakte als Stecker/Buchse-Kontaktpaare ausgebildet. Hierdurch lassen sich die Module besonders leicht elektrisch und mechanisch miteinander verbinden. In einer Ausgestaltung ist zudem eine Verriegelung der Module zur Verhinderung eines unbeabsichtigten Voneinander-Lösens vorgesehen, wobei die ersten und dritten Verbindungskontakte sowie zweiten und vierten Verbindungskontakte speziell zur gegenseitigen mechanischen Verriegelung in Art eines Bajonettverschlusses ausgebildet sind. Im Rahmen der Erfindung sind aber auch andere mechanische Fixierungs- bzw. Verriegelungsmittel einsetzbar, darunter auch solche, die nicht zwischen den einzelnen Verbindungskontakten wirken, sondern zwischen den Modulen als Ganzes.

In einer weiteren zweckmäßigen Ausführung weisen die Module proximaler Endabschnitt und Leitungskörper ein durchgehendes zentrales Lumen für einen Führungsdraht oder Mandrin auf. Typischerweise wird auch ein Teil der Längserstreckung des Moduls distaler Endabschnitt ein solches Lumen aufweisen, damit der beim Einführen eingesetzte Führungsdraht oder Mandrin auch in dieses ein Stück eindringen und somit auch die Spitze der Elektrodenleitung gezielt geführt werden kann.

In einer Ausgestaltung des erfindungsgemäßen Satzes von Elektrodenleitungs-Modulen umfasst dieser mehrere Module Leitungskörper mit unterschiedlicher Länge und/oder alternativ an das Modul Leitungskörper anschließbare Module proximaler Endabschnitt mit verschiedenen Geräteanschlusskontakten und/oder mehrere alternativ an das Modul Leitungskörper anschließbare Module distaler Endabschnitt mit unterschiedlichen Ausführungen von Elektrodenpolen und/oder unterschiedlicher mechanischer/geometrischer Ausführung. Mit einem solchen Modul-Satz lassen sich, je nach Umfang seiner Ausgestaltung, praktisch sämtliche relevanten Einsatzgebiete der Elektrodenleitung modular abdecken.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine Gesamtansicht einer implantierbaren Elektrodenleitung, angeschlossen an einen Herzschrittmacher,
- Fig. 2: drei verschiedene Module proximaler Endabschnitt eines Ausführungsbeispiels der erfindungsgemäßen Elektrodenleitung,
- Fig. 3: zwei Module Leitungskörper eines Ausführungsbeispiels der erfindungsgemäßen Elektrodenleitung,
- Fig. 4: verschiedene Sub-Module zur Bildung eines Moduls Leitungskörper eines Ausführungsbeispiels der erfindungsgemäßen Elektrodenleitung,
- Fig. 5: drei Module distaler Endabschnitt eines Ausführungsbeispiels der erfindungsgemäßen Elektrodenleitung,
- Fig. 6: schematische Darstellungen zur Verdeutlichung der Leiterseil- und Verbindungskontakt-Konfiguration an einer Modul-Verbindungsstelle eines Ausführungsbeispiels der erfindungsgemäßen Elektrodenleitung,
- Fig. 7: perspektivische schematische Darstellungen einer Ausführungsform der ersten und dritten bzw. zweiten und vierten Verbindungskontakte eines Ausführungsbeispiels der erfindungsgemäßen Elektrodenleitung, und
- Fig. 8: eine schematische Längsschnittdarstellung eines Verbindungskontakt-Bereiches eines Ausführungsbeispiels der erfindungsgemäßen Elektrodenleitung.

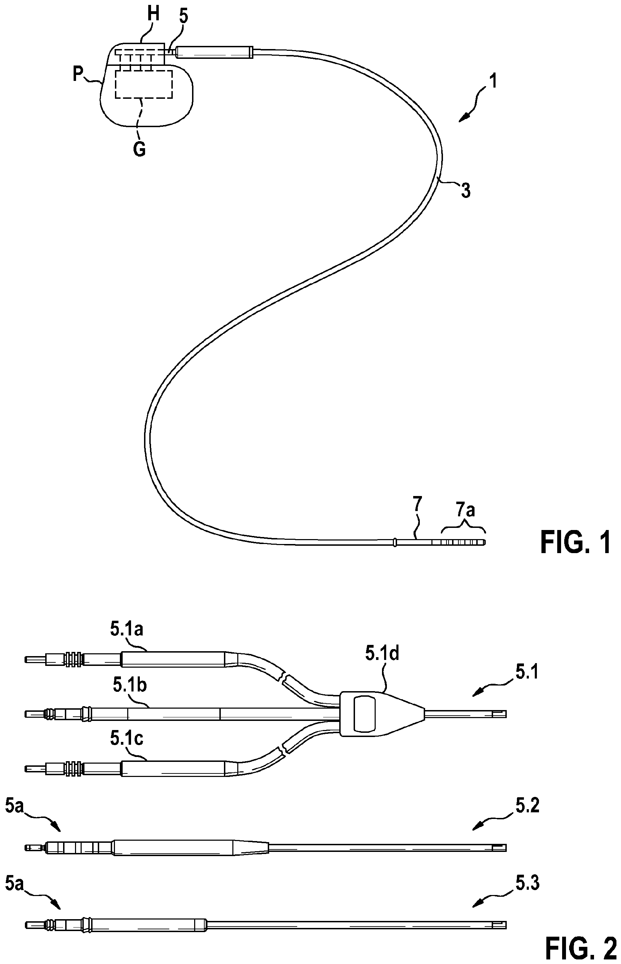

Fig. 1 zeigt eine Gesamtansicht einer modular aufgebauten Elektrodenleitung 1, die ein Modul Leitungskörper 3, ein Modul proximaler Endabschnitt (Stecker) 5 und ein Modul distaler Endabschnitt (Elektrodenpole) 7 umfasst. Mit dem Modul proximaler Endabschnitt 5 ist die Elektrodenleitung 1 an den Header H eines Herzschrittmachers P angeschlossen. Sie liefert über mehrere Elektrodenpole 7a im Modul distaler Endabschnitt 7 Stimulationsimpulse, die in einem Impulsgenerator G des Herzschrittmachers P erzeugt werden, an das (nicht gezeigte) Herz eines mit dem Schrittmacher versorgten Patienten.

Fig. 2 zeigt drei verschiedene Module proximaler Endabschnitt 5.1, 5.2 bzw. 5.3 zur Bildung verschiedener Ausführungen einer Elektrodenleitung 1 der in Fig. 1 gezeigten Art für verschiedene Einsatzzwecke. Während die Module proximaler Endabschnitt 5.2 und 5.3 jeweils einstückig sind und sich lediglich hinsichtlich der konkreten Ausführung eines einzelnen Anschlusssteckers 5a unterscheiden, besteht das Modul 5.1 aus drei Sub-Modulen 5.1a, 5.1b und 5.1c, mit denen die Elektrodenleitung parallel an drei verschiedene Anschlussbuchsen eines Stimulationsgerätes angeschlossen werden kann, und einem Verzweigungs-Modul 5.1d, welches die Leitungen der drei Sub-Module 5.1 a, 5.1b und 5.1c zusammenführt und eine Leiterkonfiguration bildet, die an das (hier nicht gezeigte) Modul Leitungskörper anschließbar ist.

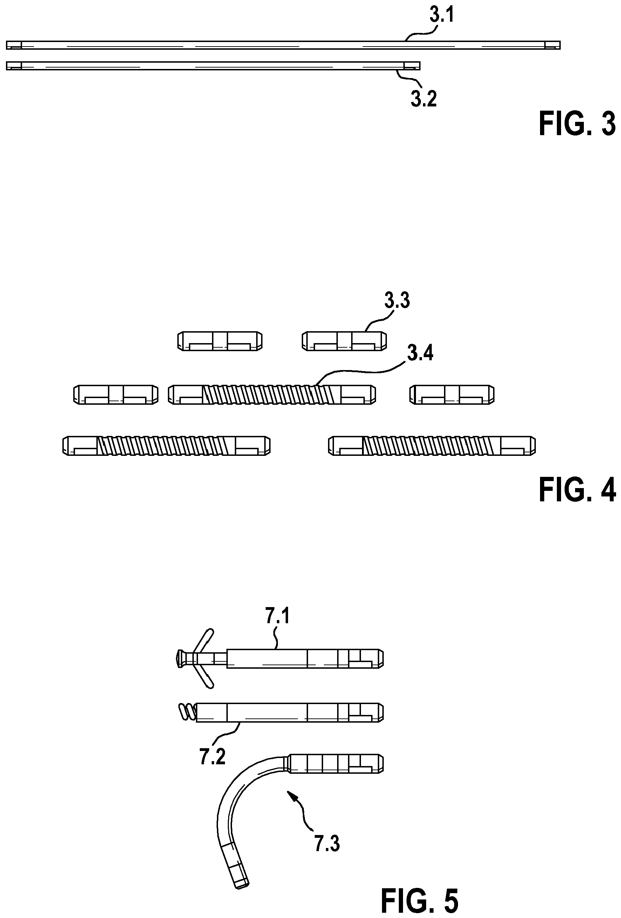

Fig. 3 zeigt zwei verschiedene Module Leitungskörper 3.1 und 3.2 eines Satzes von Elektrodenleitungs-Modulen zur Bildung verschiedener Ausführungen einer Elektrodenleitung 1 der in Fig. 1 gezeigten Art. Die Module 3.1 und 3.2 sind in ihrem Aufbau gleich und unterscheiden sich lediglich hinsichtlich ihrer Länge.

Fig. 4 zeigt verschiedene Sub-Module 3.3 und 3.4, die zur Bildung eines komplexer aufgebauten Moduls Leitungskörper genutzt werden können. Es handelt sich hierbei um ein Ringelektroden-Submodul 3.3 für Stimulationszwecke und ein Schockcoil-Submodul 3.4 für eine an einen Kardioverter anschließbare und mit diesem einsetzbare Elektrodenleitung. Es versteht sich, dass diese Sub-Module in Verbindung mit weiteren Sub-Modulen benutzt werden, mit denen ein langgestreckter Leitungskörper der Elektrodenleitung gebildet werden kann. Es könnte sich hierbei um Module bzw. Sub-Module der in Fig. 3 gezeigten Art handeln.

Fig. 5 zeigt drei verschiedene Module distaler Endabschnitt 7.1, 7.2 bzw. 7.3, die alternativ zur Bildung unterschiedlicher Stimulations-Konfigurationen an ein Modul Leitungskörper angeschlossen werden können. Während die Sub-Module 7.1 und 7.2 sich hinsichtlich der Mittel zur Verankerung der Elektrodenleitungs-Spitze am zu stimulierenden Körpergewebe (Herzgewebe) unterscheiden, nämlich im Vorhandensein einer Wiederhaken-Spitze (Modul 7.1) bzw. einer Einschraub-Spitze (Modul 7.2), hat das Modul 7.3 eine J-Form und unterscheidet sich auch in seiner Elektrodenpol-Konfiguration von den beiden anderen Modulen. Da die spezielle Ausgestaltung von Fixierungsmitteln und Elektroden-Konfigurationen eines Moduls distaler Endabschnitt im Zusammenhang mit der vorliegenden Erfindung nicht von entscheidender Bedeutung ist, wird hier von einer genaueren Beschreibung dieser Module abgesehen.

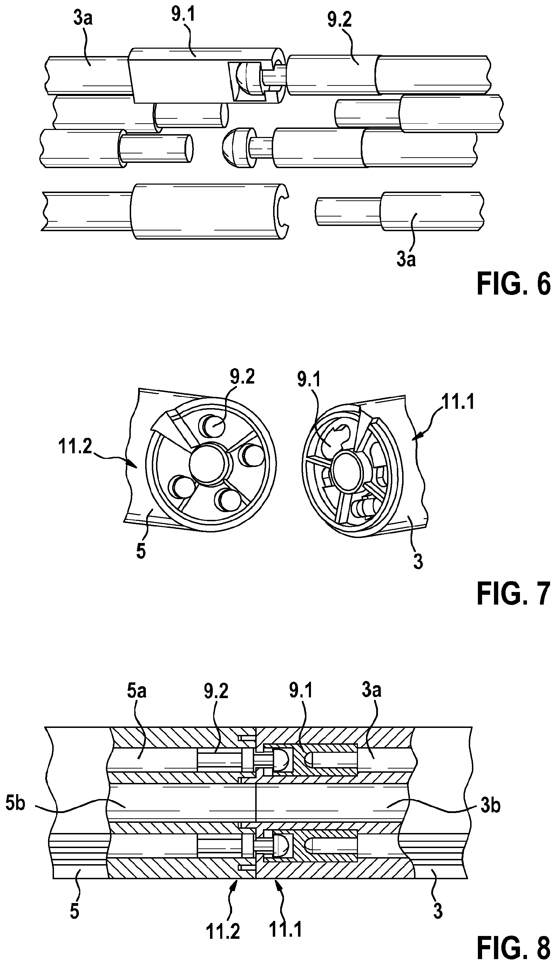

Fig. 6 zeigt schematisch einen beispielhaften Leiteraufbau einer erfindungsgemäß modular aufgebauten Elektrodenleitung, der vier Zuleitungen (Leiterseile) 3a umfasst. Die jeweils für sich genommen isolierten und darüber hinaus in einen (nicht gezeigten) isolierenden Leitungskörper eingebetteten Leiterseile 3a sind an einer Trennstelle zwischen verschiedenen Modulen oder Sub-Modulen mit Verbindungskontakten 9.1, 9.2 in Art einer Buchse-Stecker-Verbindung versehen. In der Figur ist eine der Zuleitungen in einem Zustand dargestellt, in dem die beiden in Längsrichtung aneinander anstoßenden Leiterseile 3a mit daran befestigten Verbindungskontakten 9.1 bzw. 9.2 miteinander (lösbar) verbunden sind. Die Leiterseile einer zweiten möglichen Leitungsverbindung sind mit den daran angebrachten Verbindungskontakten 9.1 bzw. 9.2, aber noch im unverbundenen Zustand gezeigt, und die verbleibenden Leiterseile sind mit freien Anschlussenden, aber ohne angebrachte Verbindungskontakte dargestellt. Es handelt sich hier also um eine synoptische schematische Darstellung, die nicht den Gebrauchszustand einer fertigen Elektrodenleitung zeigt. Es ist jedoch denkbar, dass auch bei fertiggestellten Elektrodenleitungen für bestimmte Einsatzzwecke nicht sämtliche eingebetteten Leiterseile als wirksame elektrische Verbindungen genutzt werden sollen und daher nicht mit Verbindungskontakten bestückt sind.

Fig. 7 zeigt in einer schematischen perspektivischen Darstellung die Stirnseiten zweier Module einer erfindungsgemäßen Elektrodenleitung - hier beispielhaft bezeichnet mit den Ziffern 3 für ein Modul Leitungskörper und 5 für ein Modul proximaler Endabschnitt. In Übereinstimmung mit der in Fig. 6 schematisch dargestellten Ausführung mit vier Zuleitungen haben beide Module 3, 5 jeweils vier Verbindungskontakte 9.1 bzw. 9.2, die aus der Stirnseite des entsprechenden Moduls hervorragen (Verbindungskontakte 9.2 beim Modul 5) bzw. in diese eingearbeitet sind (Verbindungskontakte 9.1 im Modul 3).

Es ist auch zu erkennen, dass die Modul-Stirnseiten eine spezielle Verriegelungs- und Dichtstruktur 11.1 bzw. 11.2 aufweisen, die in ihrer Funktion an einen Bajonettverschluss angelehnt ist. Diese Verriegelungs- und Dichtstrukturen 11.1, 11.2 ermöglichen ein sehr bequemes Verbinden der einander benachbarten Module praktisch mit einem Handgriff und sichern gleichwohl eine zuverlässige und dichte elektrische und mechanische Verbindung.

Fig. 8 zeigt die entsprechende Struktur in einer Längsschnittdarstellung genauer. Hier sind die Leiterseile des Moduls Leitungskörper 3, wie in Fig. 6, mit Ziffer 3a bezeichnet, während Zuleitungen im hieran angeschlossenen Modul proximaler Endabschnitt 5 mit Ziffer 5a bezeichnet sind. Der Aufbau der Verriegelungs- und Dichtstrukturen 11.1, 11.2, die (nicht gesondert bezeichnete) Silikon-Dichtlippen und "Schikanen" umfassen, ist hier genauer zu erkennen. Weiterhin ist zu erkennen, dass die Module 3, 5 jeweils ein zentrales Lumen 3b bzw. 5b aufweisen und diese Lumen im verbundenen Zustand der Module miteinander longitudinal ausgerichtet sind, so dass eine entsprechend aufgebaute Elektrodenleitung in üblicher Weise mit einem Führungsdraht oder Mandrin implantiert werden kann.

In Bezug auf die Figuren 6 bis 8 einerseits und die anhängenden Ansprüche sowie obigen allgemeinen Ausführungen andererseits ist auf folgendes hinzuweisen: In den Figuren sind beispielhaft zwei Arten von Verbindungskontakten gezeigt, nämlich ein "weiblicher" Kontakt 9.1 und ein "männlicher" Kontakt 9.2, mit denen eine Steckverbindung in Art einer Buchse-Stecker-Verbindung gebildet wird.

Mit diesen beiden Arten von Verbindungskontakten lassen sich die weiter oben und in den Ansprüchen erwähnten ersten bis vierten Verbindungskontakte realisieren. Mit der Bezeichnung "erste", "zweite", "dritte" und "vierte" Verbindungskontakte wird darauf hingewiesen, dass an den Enden der jeweiligen Module Verbindungskontakte einer bestimmten Art vorgesehen sind, zu denen Verbindungskontakte einer anderen Art an einem angrenzendem Modul (lösbar) passen sollen. Da bei der erfindungsgemäßen Elektrodenleitung drei Module seriell verbunden werden, reichen zur Realisierung der ersten bis vierten Verbindungskontakte zwei physisch unterschiedene Arten von Kontakten aus, es ist aber auch möglich die Erfindung tatsächlich mit vier physisch unterschiedlichen Kontakten auszuführen.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Implantierbare Elektrodenleitung (1) zur elektrischen Stimulation und/oder zum Abfühlen von Körpergewebe, mit einem distalen Endabschnitt (7), der mindestens einen Elektrodenpol (7a) und/oder einen Sensor aufweist, einem proximalen Endabschnitt (5), der mindestens einen Geräteanschlusskontakt (5a) aufweist, und einem Leitungskörper (3), der eine elektrische Zuleitung zur Verbindung des Elektrodenpols bzw. Sensors mit dem Geräteanschlusskontakt enthält,
**dadurch gekennzeichnet, dass**
der distale Endabschnitt, proximale Endabschnitt und Leitungskörper separate Module (7, 5, 3) sind, wobei das Modul distaler Endabschnitt (7) an seinem proximalen Ende mindestens einen ersten Verbindungskontakt (9.1, 9.2) hat, das Modul proximaler Endabschnitt (5) an seinem distalen Ende mindestens einen zweiten Verbindungskontakt hat und das Modul Leitungskörper (3) an seinem distalen Ende einen zum lösbaren Eingriff mit dem ersten Verbindungskontakt ausgebildeten dritten Verbindungskontakt (9.1, 9.2) und an seinem proximalen Ende einen zum lösbaren Eingriff mit dem zweiten Verbindungskontakt ausgebildeten vierten Verbindungskontakt (9.1, 9.2) hat, und die Module im verbundenen Zustand der Verbindungskontakte die Elektrodenleitung bilden.

2. Implantierbare Elektrodenleitung nach Anspruch 1, wobei das Modul distaler Endabschnitt (7) und/oder das Modul proximaler Endabschnitt (5) und/oder das Modul Leitungskörper (3) mehrere Sub-Module umfassen.

3. Implantierbare Elektrodenleitung nach Anspruch 2, wobei das Modul Leitungskörper (3) mehrere seriell verbindbare Sub-Module (3.1 - 3.4) umfasst, die insbesondere jeweils an einem Ende mindestens einen Verbindungskontakt (9.1) vom Typ des ersten Verbindungskontakts und am anderen Ende einen Verbindungskontakt (9.2) vom Typ des dritten Verbindungskontakts haben.

4. Implantierbare Elektrodenleitung nach Anspruch 3, wobei das Modul Leitungskörper (3) mindestens ein Sub-Modul (3.3, 3.4) umfasst, das einen Elektrodenpol, insbesondere eine Ringelektrode, oder eine Schock-Wendel aufweist.

5. Implantierbare Elektrodenleitung nach einem der Ansprüche 2 bis 4, wobei das Modul distaler Endabschnitt (7) mehrere seriell verbindbare Sub-Module umfasst, von denen alle, mit Ausnahme des das distale Ende der Elektrodenleitung bildenden Sub-Moduls, insbesondere an einem Ende einen Verbindungskontakt vom Typ des dritten Verbindungskontakts und einem anderen Ende einen Verbindungskontakt vom Typ des ersten Verbindungskontakts haben.

6. Implantierbare Elektrodenleitung nach einem der Ansprüche 2 bis 5, wobei das Modul proximaler Endabschnitt (5) mehrere Sub-Module (5.1a - 5.1c) mit je mindestens einem Geräteanschlusskontakt (5a) und einem Verbindungskontakt (9.1, 9.2) vom Typ des zweiten Verbindungskontakts, sowie ein Verzweigungs-Modul (5.1d) umfasst, das den zweiten Verbindungskontakt (9.1, 9.2) trägt und insbesondere mehrere Verbindungskontakte vom Typ des vierten Verbindungskontakts hat.

7. Implantierbare Elektrodenleitung nach einem der vorangehenden Ansprüche, wobei das Modul distaler Endabschnitt (7) eine Mehrzahl von ersten Verbindungskontakten (9.1, 9.2) hat, das Modul proximaler Endabschnitt (5) eine Mehrzahl von zweiten Verbindungskontakten (9.1, 9.2) hat und das Modul Leitungskörper (3) eine Mehrzahl von dritten Verbindungskontakten (9.1, 9.2) und eine Mehrzahl von vierten Verbindungskontakten (9.1, 9.2) hat und die Anzahl der Verbindungskontakte aller Typen übereinstimmt.

8. Implantierbare Elektrodenleitung nach einem der vorangehenden Ansprüche, wobei das Modul Leitungskörper (3) eine Mehrzahl von Leiterseilen (3a) umfasst, an denen der oder jeder dritte und vierte Verbindungskontakt (9.1, 9.2) durch Crimpen oder Laserschweißen angebracht ist/sind.

9. Implantierbare Elektrodenleitung nach Anspruch 8, wobei im Modul Leitungskörper (3) nur ein Teil der Leiterseile (3a) mit einem dritten und vierten Verbindungskontakt (9.1, 9.2) versehen ist.

10. Implantierbare Elektrodenleitung nach einem der vorangehenden Ansprüche, wobei die ersten und hierin eingreifenden dritten Verbindungskontakte (9.1, 9.2) sowie die zweiten und hierin eingreifenden vierten Verbindungskontakte (9.1, 9.2) als Stecker/Buchse-Kontaktpaare ausgebildet sind.

11. Implantierbare Elektrodenleitung nach Anspruch 10, wobei die ersten und dritten Verbindungskontakte (9.1, 9.2) sowie zweiten und vierten Verbindungskontakte (9.1, 9.2) zur gegenseitigen mechanischen Verriegelung in Art eines Bajonettverschlusses ausgebildet sind.

12. Implantierbare Elektrodenleitung nach einem der vorangehenden Ansprüche, wobei die Module proximaler Endabschnitt und Leitungskörper (5, 3) ein durchgehendes zentrales Lumen (5b, 3b) für einen Führungsdraht oder Mandrin aufweisen.

13. Satz von Elektrodenleitungs-Modulen (3, 5, 7) zur Bildung einer implantierbaren Elektrodenleitung (1) nach einem der vorangehenden Ansprüche, umfassend mindestens ein Modul distaler Endabschnitt (7), mindestens ein Modul proximaler Endabschnitt (5) und mindestens ein Modul Leitungskörper (3).

14. Satz von Elektrodenleitungs-Modulen nach Anspruch 13, wobei das Modul distaler Endabschnitt (7) und/oder das Modul proximaler Endabschnitt (5) und/oder das Modul Leitungskörper (3) eine Mehrzahl von Sub-Modulen (3.1 - 3.4; 5.1a - 5.1 d) umfasst.

15. Satz von Elektrodenleitungs-Modulen nach Anspruch 13 oder 14, der mehrere Module Leitungskörper (3.1, 3.2) mit unterschiedlicher Länge und/oder alternativ an das Modul Leitungskörper anschließbare Module proximaler Endabschnitt (5.1 - 5.3) mit verschiedenen Geräteanschlusskontakten (5a) und/oder mehrere alternativ an das Modul Leitungskörper anschließbare Module distaler Endabschnitt (7.1 - 7.3) mit unterschiedlichen Ausführungen von Elektrodenpolen (7a) und/oder unterschiedlicher mechanischer/geometrischer Ausführung umfasst.
